# EUROPEAN PATENT APPLICATION

(11) **EP 2 711 432 A1**
(43) Date of publication of application: **26.03.2014**
(21) Application number: 12382370.0
(22) Date of filing: 21.09.2012
(51) Int. Cl.: C12Q 1/68

(54) **Method for detection of BRAF and PI3K mutations**

(71) Applicant: Genomica S.A.U., 28823 Coslada, Madrid (ES)
(72) Inventor: Villahermosa, María Luisa, 28823 Coslada, Madrid (ES); Moscoso del Prado, Juan, 28823 Coslada, Madrid (ES)
(74) Representative: Gascón Escobar, Irene

(57) **Abstract**

The present invention is based on a detection method of the BRAF mutations V600E and V600K, and of the PI3K mutations E542K, E545D, E545K and H1047R, in a sample susceptible of containing one or more of such mutations, based on amplification of the sample with the primers of the present invention.

Further, the present invention relates to (i) a kit which comprises, amongst its components, amplification reagents including one or more of the primers of the present invention; (ii) the primers themselves; and (iii) use of the method, kit and primers of above, for the diagnosis/ prognosis of a pathological condition in a patient, particularly, of cancer.

## Description

### Background of the invention.

B-raf (or BRAF) is a part of the Ras/Raf/MEK/MAP signal transduction pathway and plays a role in regulating the MAP Kinase/ ERK signalling pathway. Mutations in this gene have been associated with various cancers such as colorectal cancer (CRC), non small cell lung cancer (NSCLC), malignant melanomas and adenocarcinomas. Oncogenic mutations in BRAF, nearly all of which are the V600E mutation, have been reported in colon cancer (Davies H, et al., 2002, Nature 417:949-54; Rajagopalan H, et al., 2002, Nature 418:934). The V600E mutation is located on exon 15 of the BRAF gene: At position 1799 of the BRAF coding sequence, a T is changed to an A, which results in the change from a valine (V) present in the wildtype BRAF protein, to a glutamine (E) in the protein corresponding to the mutated gene. V600K mutation (1798-1799 GT>AA), less abundant, has also been detected, and constitutes the second most abundant mutation in pathologies such as melanoma (Rubinstein et al., 2010, Journal of Translational Medicine 8, No pp. given). Other identified BRAF gene mutations are V600D, V600M and V600A.

The phosphatidylinositol 3-kinase (PI3K) pathway plays an important role in many cellular processes including cell proliferation, adhesion, survival and motility. Disregulation of this pathway has been observed in many types of human malignancy and has commonly been associated with genetic alterations in components of the pathway. Such genetic alterations include activating mutations in the p110α subunit of PI3K, PIK3CA (Hurst et al., 2009, BMC Research Notes 2:66). Most mutations of PI3K occur either in exon 9 of PIK3CA, which codes for the helical domain, or exon 20 of PIK3CA, which codes for the kinase domain (Engelman, 2009, Nat. Rev. Cancer 9: 550-562). Four frequent PI3K mutations in above-mentioned exons are E542K, E545D, E545K (the three of them located in exon 9) and H1047R (exon 20).

Recent publications suggest that mutations in BRAF and PI3K may confer resistance to anti-EGFR therapy (Lurkin et al., 2010, PLoS ONE, 5, (1); De Roock et al., 2010, Lancet Oncol. 11: 753-62; De Roock et al., 2011, Lancet Oncol. 12: 594-603; Bardelli & Sienna, 2010, J Clin Oncol; 28(7): 1254-1261). In particular, mutations in BRAF were shown to impair response to panitumumab or cetuximab in patients with mCRC (Di Nicolantonio F et al., 2008, J. Clin. Oncol. 26:5705-5712), the presence of mutated BRAF representing a negative prognostic factor for mCRCs. Current data also suggest that the evaluations of BRAF and PI3K alterations could be useful for selecting patients with mCRC who are unlikely to respond to anti-EGFR-targeted antibodies (Bardelli & Sienna, 2010, J Clin Oncol; 28(7): 1254-1261).

There is a high demand for methods of detection of mutations of the PI3K and BRAF genes. Recently, a method for the simultaneous detection of gene mutations that are relevant for response to anti-EGFR treatment has been disclosed (Lurkin et al., 2010, PLoS ONE, 5, (1)). The method includes analysis of PI3K mutations in exons 9 and 20, as well as analysis of BRAF mutations in exon 15. Basically, two multiplex PCRs were designed; the first one comprising a pair of primers for amplification of BRAF exon 15; and the second one comprising two pairs of primers for amplification of PI3K exons 9 and 20. Subsequently, the obtained PCR products are subjected to purification, denaturation and incubation with probes which hybridise with one of the strands of their corresponding amplification product, in a position adjacent to the mutation site. Extension reactions are then performed in a thermal cycler, wherein only the ddNTP complementary to the mutation that is present in the sample is incorporated into the extension product, and detected with an automatic sequencer.

Other mutation detection methods in these genes, include Real-Time ARMS (amplification refractory mutation system) assays, such as those disclosed in Ellison et al., 2010, Journal of Experimental & Clinical Cancer Research 29: 132.

ARMS is a method for detecting point mutations, based on the principle of allele-specific priming of PCR amplification (EP0332435; Newton et al., 1989, Nucleic Acid Research 17, 2503-2516). This system is based on a strategy wherein each oligonucleotide primer or primer is designed so that it only functions as a primer for the PCR when it anneals to its corresponding specific target DNA sequence. The technique requires that the terminal 3'-nucleotide of the PCR primer be allele specific. This implies that the terminal 3'-nucleotide corresponds to that of the point mutation. Thus, the primer is designed in two forms: The "normal" form, which is refractory to PCR amplification of the "mutant" DNA template, and the "mutant" form, which is refractory to PCR amplification of the "normal" DNA.

In some instances, a single 3'-mismatched base does not completely prevent the nonspecific extension of the oligonucleotide primer when having as target the DNA corresponding to another point mutation, and amplification proceeds. In such cases, deliberate introduction of a mismatch near the 3' end of the allele-specific appropriate primer (at the second, third, or even fourth nucleotide from the 3' end of the primer) allows to enhance the specificity of the primer.

The ARMS technique involves that at least two PCRs may take place in one reaction mixture, each corresponding to amplification with one of the ARMS primers. Any ARMS primer further requires a second primer (usually referred to as the "common primer", and that will be hereafter called "amplification primer") to generate the allele-specific product.

The presence of the specific target sequence in a sample is revealed by visualization of the product after agarose gel electrophoresis and ethidium bromide staining. Alternatively, the results may be analysed in a real-time, closed-tube format by incorporating fluorescent probes such as Taqman, Scorpions, Molecular beacons or intercalating fluorescent dyes such as Yo-Pro or Sybr green.

The state of the art often deals with the issue of detecting BRAF mutations V600E and V600K, and PI3K mutations E542K, E545D, E545K and H1047R. However, to the best of our knowledge, none of the existing detection methods of state of the art, allows detection of any amplification products obtained from these mutations, through their hybridization with specific probes. This is due to the fact that any probe that might be designed for hybridization with any amplification product corresponding to one of the mutations of one of these genes, would also un-specifically bind to the amplification products of nearby mutations in the same gene, due to the sequence similarity between them. Thereby, detection methods of BRAF and PI3K mutations of the state of the art have the drawback that detection of any amplification products obtained cannot be carried out through hybridization of the former with mutation-specific probes, and, in particular, with microarrays containing such probes.

This drawback applies both to the Multiplex ARMS amplification approach, as well as to the individual ARMS amplification approach, wherein specific amplification products from each mutation are obtained in independent reaction vessels.

Another associated problem is that the BRAF and PI3K mutations that may present as prognostic factors for tumour staging, metastasis, evolution, cellular heterogeneity, or allelic heterogeneity, are often to be found in samples in low abundance with respect to the wild type form. And, although many diagnostic methods are available for mutation detection, most cannot accurately detect low-abundance mutations. Sanger sequencing is the gold standard for mutation identification though it may only detect mutations in abundances above approximately 20% (Ogino et al., 2005, J. Mol. Diagn. 7: 413-421; Li et al., 2008, Nat. Med. 14: 579-584).

It is therefore highly desirable to provide for a method that, differently to the methods of the State of the Art, allows to specifically detect any amplification product, obtained from any of the BRAF mutations V600E and V600K, and PI3K mutations E542K, E545D, E545K and H1047R present in a sample, by hybridization of the corresponding amplification products with target-specific probes. It is also desirable that the method allows detection of mutations present in low percentages within the sample. The invention described herein is thus aimed at providing a robust and reliable method for detecting BRAF and PI3K mutations, and thus at mitigating the shortcomings in the prior art.

### Summary of the invention.

The problem to be solved by the present invention is the provision of a detection method of any one of the 2 BRAF mutations V600E and V600K, or of any one of the 4 PI3K mutations E542K, E545D, E545K and H1047R present in a sample, through hybridization of any amplification product obtained from any of these mutations, with target-specific probes.

The solution, which is used in the different methods and aspects described herein, is based on amplification of one or more of the 2 BRAF mutations V600E and V600K or of one or more of the of the 4 PI3K mutations E542K, E545D, E545K and H1047R present in a sample, with one or more ARMS primers of the present invention.

The ARMS primers of the present invention are characterised in that each primer has a total length of from 36 to 50 nucleotides, their 3' end being designed in accordance with the ARMS amplification method (amplification refractory mutation system, method for detecting point mutations based on the principle of allele-specific priming of PCR amplification), and this 3' end constituting a target specific sequence selected from SEQ ID Nº 1 and SEQ ID Nº 2, respectively, in the case of BRAF V600E and V600K mutations, and SEQ ID Nº 12, SEQ ID Nº 13, SEQ ID Nº 14, and SEQ ID Nº 15, respectively, in the case of PI3K mutations E542K, E545D, E545K and H1047R, each primer further comprising a different non-target specific 5' tag sequence of from 15 to 20 nucleotides.

Thus, the ARMS primer used in the method of the present invention comprises a target specific sequence in the 3' end, which is capable of hybridising to the target nucleic acid in an allele specific way. This primer also comprises a 5' sequence which is not target specific and thus not complementary to the target nucleic acid. This sequence is a tag sequence which is useful in detecting the amplification product through subsequent hybridisation with a probe specifically designed to hybridise to the region of the amplification product complementary to the tag sequence. This allows the specific detection of the amplification product by the methods described herein.

The method of the present invention allows to specifically detect any amplification product, obtained from any of the 2 BRAF mutations V600E and V600K or PI3K mutations E542K, E545D, E545K and H1047R present in a sample, through hybridization of such product with one or more probes that specifically hybridize to it in the region corresponding to the tag provided by the specific primer.

The 5' tag sequences of the different primers, are all different from the others. This difference is what makes possible the specific binding of the different amplification products to their corresponding probes, for any probe specific for one of the amplification products must have a nucleotide sequence able to hybridise at least to the region in the product corresponding to the corresponding tag.

Throughout the present patent specification these mutation-specific primers designed in their 3' end in accordance with the ARMS amplification method, will be named "ARMS primers". Additionally, the primer or primers that combine with the ARMS primers for target DNA amplification, also usually referred to as the common primers, will be hereafter named "amplification primers".

The specific ARMS primers for the BRAF and PI3K mutations of the present invention, can be depicted as indicated in Table 1.

| Table 1: | |
|---|---|
| **BRAF V600E** | **5' tag1-** GGTGATTTTGGTCTAGCTTCAGA **3',** |
| **BRAF V600K** | **5' tag2-** GGTGATTTTGGTCTAGCTACTAA **3',** |
| **PI3K E542K** | **5' tag3-** AAGCAATTTCTACACGAGATCCTCTGTCTA **3',** |
| **PI3K E545D** | **5' tag4-** CCTCTCTCTGAAATCAGTGAT **3',** |
| **PI3K E545K** | **5' tag5-** GATCCTCTCTCTGAAATCAGTA **3',** |
| **PI3K H1047R** | **5' tag6-** GAAACAAATGAATGATGCTCGT **3',** |

Tag1, tag2, tag3, tag4, tag5 and tag6, are nucleotide sequences of 15 to 20 nucleotides, and most preferably, of 17 to 19 nucleotides in length, all being different from each other. In other words, there is no substantial homology between the tag sequences. By no substantial homology is meant less than 50% (i.e. two tag sequences must not share more than 50% of their nucleotides).

In a preferred embodiment, the GC-content of the tag is 11-71%, most preferably, 40-60%. In another preferred embodiment, the Tm of the tag is of 39-70º C. Preferably, the total length of the ARMS primer is of between 39 and 47 nucleotides.

Particularly preferred ARMS primers are those of SEQ ID Nº 3 and SEQ ID Nº 4, corresponding to BRAF mutations V600E and V600K, respectively, and those of SEQ ID Nº 16, SEQ ID Nº 17, SEQ ID Nº 18 and SEQ ID Nº 19, corresponding to PI3K mutations E542K, E545D, E545K and H1047R, respectively.

Further to the ARMS primers, the amplification mixture of the present invention comprises one or more amplification primers, which combine with the ARMS primers to generate the amplification products.

The method of the present invention thus comprises contacting a test sample comprising nucleic acids with one or more diagnostic ARMS primers specific for one or more of the of the BRAF or PI3K mutations of above, in the presence of one or more amplification primers, appropriate nucleotide triphosphates and an agent for polymerisation, and under the appropriate conditions, such that each diagnostic ARMS primer is extended only when its corresponding mutation is present in the sample; the presence or absence of each mutation is detected by reference to the presence or absence of the corresponding diagnostic primer extension product. Preferably, the presence or absence of any such product will be determined through hybridization of any amplification product obtained, with tag-specific probes.

The methods of amplification and detection of BRAF and PI3K mutations of the present invention differ from those of the state of the art in the use of the ARMS primers described herein. Amplification with these primers allows subsequent specific hybridization of the amplification products with sequence-specific probes.

In particular, amplification of one or more of the 2 BRAF mutations V600E and V600K, or of one or more of the 4 PI3K mutations E542K, E545D, E545K and H1047R, with one or more of the corresponding ARMS primers of the present invention displayed in Table 1, allows, in contrast to the BRAF and PI3K detection methods of the state of the art, to detect any resulting amplification product through hybridization with sequence-specific probes. Preferably, target-specific probes may be provided in a microarray.

The key fact that allows the specific binding between the amplification products and their corresponding probes is that the detection probes specifically hybridize to their corresponding amplification product, in the region corresponding to tag1, tag2, tag3, tag4, tag5 or tag6 provided by the corresponding ARMS primer. This tag region has no substantial homology between the different primers. Thereby, specificity of the hybridization between any amplification product and its corresponding probe (s) is achieved.

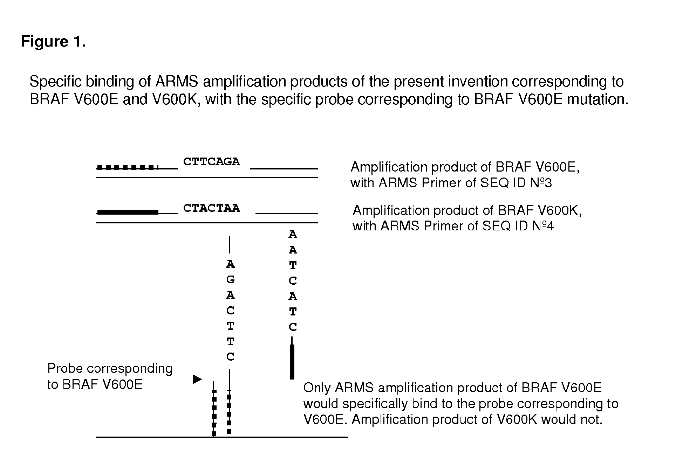

For an easier understanding of the method of the present invention, Figure 1 displays a scheme of it. As an example, detection of BRAF mutation V600E has been selected. The method of the present invention constitutes a relevant advantage vs the methods of the state of the art, the reason being that the specific hybridization of the amplification products with specific probes results in a one-step, much simpler detection method than the methods of the state of the art for detection of BRAF and PI3K mutations.

Lurkin et al., 2010, PLoS ONE, 5, (1), discloses a detection method based on DNA amplification followed by probe hybridization. In particular two multiplex PCRs were designed, the first allowing detection of BRAF exon 15, and the second allowing detection of PI3K exons 9 and 20. The amplification products obtained for each gene unspecifically hybridise to a probe, the probe hybridising with the corresponding products in a position adjacent to the mutation site of interest. The specific detection is only achieved upon extension of the hybrid formed by the probe and the amplification product. The obtained products are processed and subsequently analysed on an automatic sequencer, with the fluorescent label on the incorporated ddNTP indicating the mutation presence or absence. Thereby, in Lurkin *et al.* the amplification products obtained unspecifically bind to the probe provided, and it is only upon extension of the hybrid formed by the probe and the amplification product that the specific detection will be achieved. This means that at least an additional reaction to that of amplification is required for the detection to be achieved.

Differently, within the method of the present invention, detection takes place through the straightforward hybridization of any amplification product obtained to its sequence-specific probe.

The skilled person faced with the problem of detecting BRAF or PI3K mutations present in a sample, through hybridization of any amplification product obtained from any of these mutations, with target-specific probes, and taking a look at Lurkin *et al.,* would only have knowledge of the primers enclosed therein, originally described in Hurst et al., 2009, BMC Res. Notes 2, 66. These primers have a length of from 20 to 21 nucleotides, their sequences being 100% identical to the sequences of the genes that are to be detected with them. The criteria used to design these primers was that the PCR amplification product to be obtained should cover the position of the mutation, and that the single strands corresponding to the PCR products obtained should contain as little potential secondary structure as possible. Nowhere within the disclosure of Lurkin *et al.* or of Hurst *et al.,* is to be found a reference to primers 36 to 50 nucleotides-long, nor that would comprise a sequence not perfectly identical to that of the gene to be detected with them. Thereby, no pointer to the 36 to 50 nucleotide-long primers of the present invention, nor to the possibility of designing primers comprising sequences not identical to their BRAF or PI3K targets, is to be found in the state of the art.

The method of the present invention, also allows Multiplex PCR amplification with two or more ARMS primers of the present invention. Additionally, the method of the present invention allows detection of mutations that are present in the sample in a low percentage.

One of the aspects of the present invention corresponds to a method for detecting one or more BRAF mutations selected from V600E and V600K, or one or more PI3K mutations selected from E542K, E545D, E545K and H1047R, wherein the method comprises the steps of:
- subjecting a test sample comprising nucleic acids to amplification with an amplification mixture comprising one or more ARMS primers, characterised in that each ARMS primer has a total length of from 36 to 50 nucleotides, and comprises a 3' target specific sequence selected from SEQ ID Nº 1, SEQ ID Nº 2, SEQ ID Nº 12, SEQ ID Nº 13, SEQ ID Nº 14 and SEQ ID Nº 15, respectively,
   each ARMS primer further comprising a different non-target specific 5' tag sequence of from 15 to 20 nucleotides,
   the amplification mixture further comprising one or more amplification primers; and
- detecting any amplification product obtained, through hybridisation of any such product with one or more probes, wherein each probe specifically hybridises with the region in the amplification product corresponding to the 5' tag sequence of the corresponding ARMS primer.

Another aspect of the present invention relates to a kit for detecting one or more BRAF mutations selected from V600E and V600K, or one or more PI3K mutations selected from E542K, E545D, E545K and H1047R in a test sample comprising nucleic acid, wherein said kit comprises one or more mixtures of reagents for nucleic acid amplification, each mixture comprising:
- one or more ARMS primers characterised in that each primer has a length of from 36 to 50 nucleotides, comprising a 3' target specific sequence selected from SEQ ID Nº 1, SEQ ID Nº 2, SEQ ID Nº PIK1, SEQ ID Nº 12, SEQ ID Nº 13, SEQ ID Nº 14 and SEQ ID Nº 15, each primer further comprising a different non-target specific 5' tag sequence of from 15 to 20 nucleotides, and
- one or more amplification primers,
   the kit further comprising a microarray wherein one or more probes are immobilised, each probe specifically hybridising to the region in the corresponding ARMS product complementary to the 5' tag sequence of the corresponding ARMS primer.

Another aspect of the present invention corresponds to an amplification method of a nucleic acid corresponding to one or more BRAF mutations selected from V600E and V600K, or to one or more PI3K mutations selected from E542K, E545D, E545K and H1047R, wherein the method comprises contacting a sample containing nucleic acids with an amplification mixture comprising one or more ARMS primers characterised in that each ARMS primer has a total length of from 36 to 50 nucleotides, and comprises a 3' target specific sequence selected from SEQ ID Nº 1, SEQ ID Nº 2, SEQ ID Nº 12, SEQ ID Nº 13, SEQ ID Nº 14 and SEQ ID Nº 15, respectively,
each ARMS primer further comprising a different non-target specific 5' tag sequence of from 15 to 20 nucleotides,
the amplification mixture further comprising one or more amplification primers.

Further to the ARMS and amplification primers, the amplification mixture also comprises additional reagents for nucleic acid amplification, such as a DNA polymerase and dNTPs.

Preferably, the 5' tag may be selected from one of the following sequences SEQ ID Nº 6, SEQ ID Nº 7, SEQ ID Nº 22, SEQ ID Nº 23, SEQ ID Nº 24 and SEQ ID Nº 25. Probes of the present invention may thus comprise a sequence selected from SEQ ID Nº 6, SEQ ID Nº 7, SEQ ID Nº 22, SEQ ID Nº 23, SEQ ID Nº 24 and SEQ ID Nº 25, and may contain additional nucleotides to those specific to the tag region.

Preferably, one or more probes with which the amplification products are contacted have a length of from 15 to 45 nucleotides, the region of the probe which specifically hybridizes to the region in the product corresponding to the 5' tag sequence of the ARMS primer, having a length of from 15 to 20 nucleotides. Even more preferably, one or more probes comprise sequences selected from SEQ ID Nº 6, SEQ ID Nº 7, SEQ ID Nº 22, SEQ ID Nº 23, SEQ ID Nº 24 and SEQ ID Nº 25.

Preferably, the probes of the present invention are selected from SEQ ID Nº 8 (BRAF V600E), SEQ ID Nº 9, SEQ ID Nº 10, SEQ ID Nº 11 (BRAF V600K), SEQ ID Nº 26 (PI3K E542K), SEQ ID Nº 27 (PI3K E545D), SEQ ID Nº 28 (PI3K E545K) and SEQ ID Nº 29 (PI3K H1047R).

The probes may be immobilised on a solid support. The set of probes of the microarray and solid support may further comprise one or more control probes.

Another aspect of the present invention corresponds to an ARMS primer of from 36 to 50 nucleotides, characterized in that said primer comprises a 3' BRAF target specific sequence selected from SEQ ID Nº 1 and SEQ ID Nº 2, or a 3' PI3K target specific sequence selected from SEQ ID Nº 12, SEQ ID Nº 13, SEQ ID Nº 14 and SEQ ID Nº 15, as well as a non-target specific 5' tag sequence of from 15 to 20 nucleotides. Preferably, the 5' tag sequence is 17 to 19 nucleotides long. In a preferred aspect, the one or more ARMS primers are selected from the group comprising SEQ ID Nº 3, SEQ ID Nº 4, SEQ ID Nº 16, SEQ ID Nº 17, SEQ ID Nº 18 and SEQ ID Nº 19.

Yet another aspect of the present invention corresponds to the use of the detection and amplification methods, the kit, or the ARMS primers as described herein, for diagnosis and/or prognosis of a pathologic condition in a patient. Preferably, the pathologic condition is cancer. Most preferably, colorectal cancer.

Another aspect relates to a method for detecting/diagnosing cancer in a patient comprising a detection or amplification method as described herein. Additionally, a further aspect of the present invention corresponds to prediction of response of a patient to therapy with anti-EGFR antibodies, through performance of the methods and kit of the present invention.

### Brief description of the drawings.

Figure 1.
   Figure 1 is a scheme that shows the specific hybridization of the amplification product obtained with an ARMS primer of the present invention corresponding to BRAF mutation V600E, and the corresponding specific probe. As it is indicated in the figure, only the ARMS product of BRAF V600E mutation binds specifically to the probe corresponding to V600E, while the ARMS product of V600K does not. Similarly, only the amplification product of V600K binds specifically to the probe corresponding to V600K, while the ARMS product of V600E does not (not shown).
Figure 2.

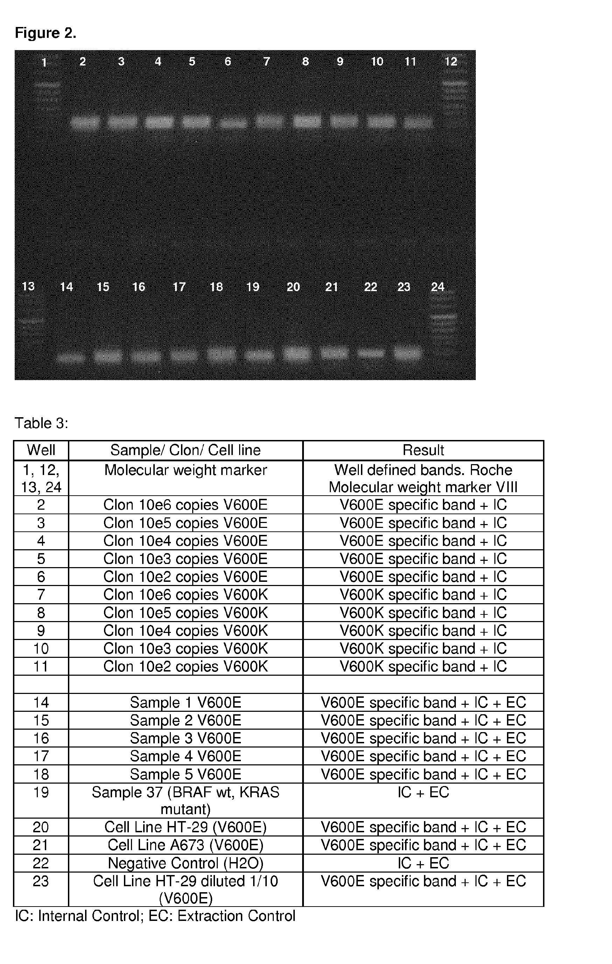

   Figure 2 displays visualization, in a 2% agarose gel, of the products of the Multiplex ARMS amplification of the BRAF mutations V600E and V600K, carried out with the ARMS and amplification primers of the present invention, according to Example 1 (Multiplex 1), and the indicated samples/ cell lines/ clones corresponding to the indicated BRAF mutations V600E or V600K.
Figure 3.

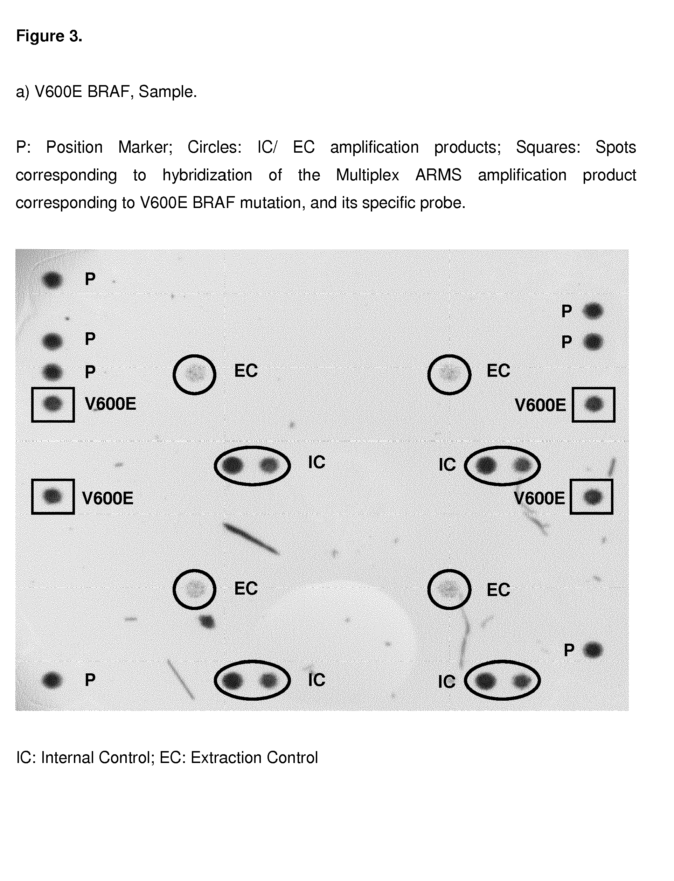

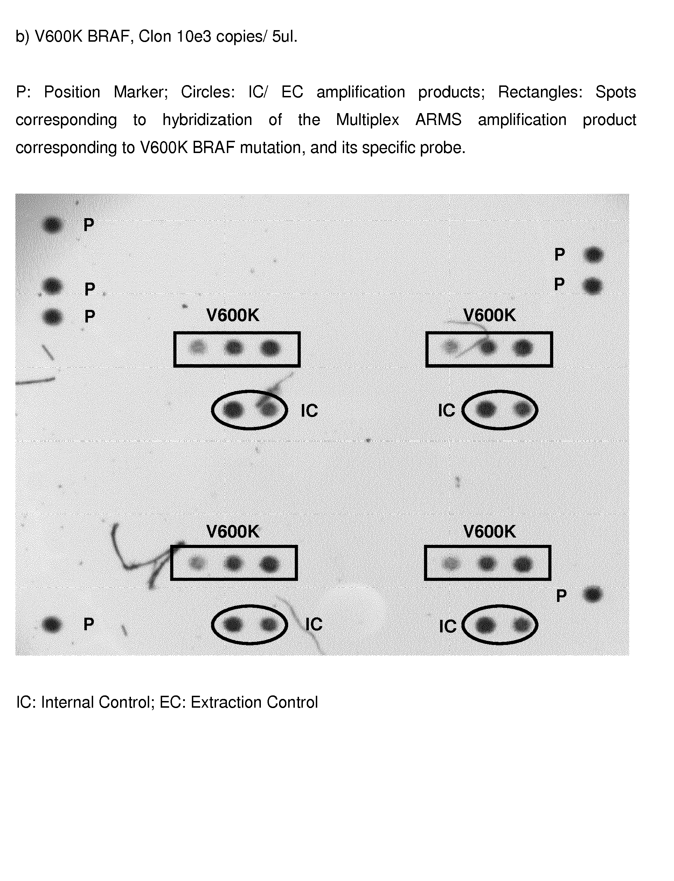

   Figure 3 displays visualization of the hybridization with a microarray comprising the probes of the present invention, of the products that result from Multiplex ARMS amplification according to Example 1 of the present invention. (a) Sample comprising V600E BRAF mutation; (b) Clon of 10e3 copies/ 5ul of V600K BRAF mutation.
   The spots named "P" correspond to the Position Marker; The spots surrounded by Circles, correspond to either the IC or EC amplification products; The spots surrounded by Squares (in (a)), correspond to the specific binding of the V600E BRAF ARMS amplification product with their specific probes; The spots surrounded by rectangles (in (b)), correspond to the specific binding of the V600K BRAF ARMS amplification product with its specific probes.
Figure 4.

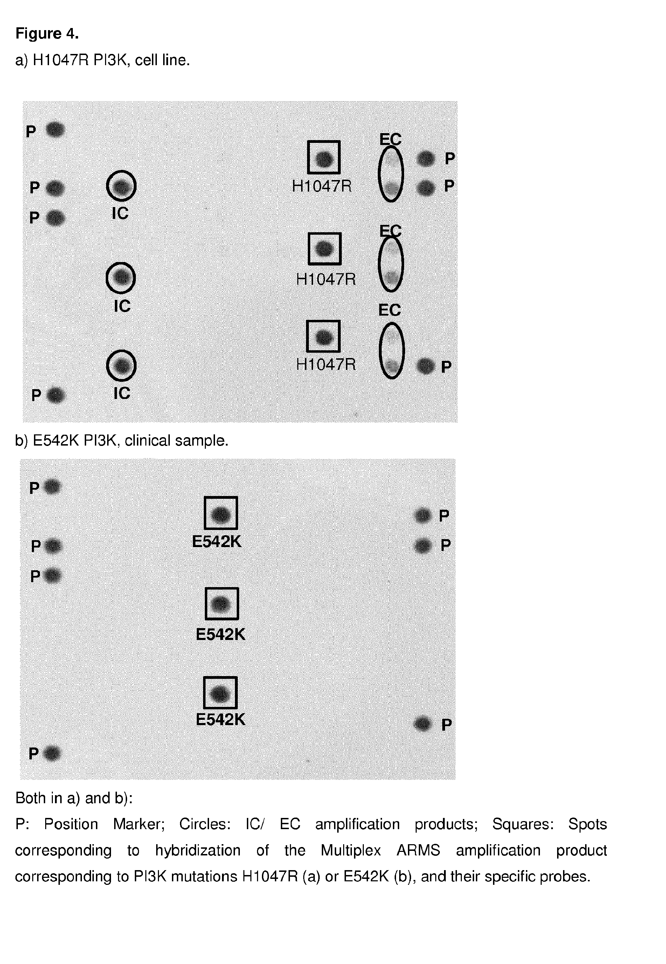

   Figure 4 displays visualization of the hybridization with a microarray comprising the probes of the present invention, of the products that result from Multiplex ARMS amplification according to Example 2 of the present invention.
   (a) Cell line HTC 116 containing H1047R PI3K mutation; (b) Clinical sample comprising E542K PI3K mutation.
   The spots named "P" correspond to the Position Marker; The spots surrounded by Circles, correspond to either the IC or EC amplification products; The spots surrounded by Squares correspond to the specific binding of the H1047R PI3K ARMS amplification product with its specific probes (in (a)) or to the specific binding of the E542K PI3K ARMS amplification product with its specific probes (in (b)).

### Detailed description of the invention.

In the following passages, different aspects of the present invention are defined in more detail. Each aspect so defined may be combined with any other aspect or aspects unless clearly indicated to the contrary. In particular, any feature indicated as being preferred or especially advantageous may be combined with any other preferred or advantageous feature or features.

A first aspect of the present invention is a method for detecting one or more BRAF mutations selected from V600E and V600K, or one or more PI3K mutations selected from E542K, E545D, E545K and H1047R, in a test sample comprising nucleic acids wherein said method comprises:
- subjecting the sample to amplification with an amplification mixture comprising one or more ARMS primers, characterised in that each primer has a total length of from 36 to 50 nucleotides, and comprises a 3' target specific sequence selected from SEQ ID Nº 1, SEQ ID Nº 2, or SEQ ID Nº 12, SEQ ID Nº 13, SEQ ID Nº 14 and SEQ ID Nº 15, respectively,
   each primer further comprising a different non-target specific 5' tag sequence of from 15 to 20 nucleotides,
   the amplification mixture further comprising one or more amplification primers, and
- detecting any amplification product obtained, through hybridization of any such product with one or more specific probes, wherein the specific probe to any BRAF or PI3K mutation specifically hybridizes with the product at least in the product region corresponding to the 5' tag sequence provided by the corresponding specific ARMS primer.

A second aspect of the present invention corresponds to a kit for detection of one or more BRAF mutations selected from V600E and V600K, or one or more PI3K mutations selected from E542K, E545D, E545K and H1047R present in a sample. Specifically, the invention relates to a kit for detecting any such mutation in a test sample comprising nucleic acid, wherein said kit comprises one or more mixtures of reagents for nucleic acid amplification, each mixture comprising:
- one or more ARMS primers characterised in that each primer has a length of from 36 to 50 nucleotides, comprising a 3' target specific sequence selected from SEQ ID Nº 1, SEQ ID Nº 2, SEQ ID Nº 12, SEQ ID Nº 13, SEQ ID Nº 14 and SEQ ID Nº 15, respectively,
   each primer further comprising a different non-target specific 5' tag sequence of from 15 to 20 nucleotides, and
- one or more amplification primers,
   the kit further comprising a microarray wherein one or more specific probes are immobilised. Each specific probe specifically hybridises to the region in the corresponding ARMS product complementary to the 5' tag sequence of the corresponding ARMS primer.

A third aspect of the present invention relates to a method for amplifying a nucleic acid in a test sample, said nucleic acid comprising one or more BRAF mutations selected from V600E and V600K, or one or more PI3K mutations selected from E542K, E545D, E545K and H1047R, wherein said method comprises contacting said sample with one or more ARMS primers characterised in that each primer has a total length of from 36 to 50 nucleotides, and comprises a 3' target specific sequence selected from SEQ ID Nº 1, SEQ ID Nº 2, SEQ ID Nº 12, SEQ ID Nº 13, SEQ ID Nº 14 and SEQ ID Nº 15, respectively,
each primer further comprising a different non-target specific 5' tag sequence of from 15 to 20 nucleotides,
the amplification mixture further comprising one or more amplification primers.

Most preferably: each ARMS primer has a total length of from 39 to 47 nucleotides; the 3' target-specific sequence has a length of from 21 to 30 nucleotides; the 5' tag sequence has a length of from 17 to 19 nucleotides; the detection probe has a length of from 18 to 38 nucleotides.

The methods and kit of the present invention imply that, further to the ARMS and amplification primers, any other additional components known by the skilled person to be necessary for nucleic acid amplification, and in particular, for PCR amplification, may also be present within the amplification mixture. Thereby, a DNA polymerase and dNTPs, may also be present within the amplification mixture.

Preferably, the amplification product is transformed into single-stranded DNA prior to hybridization with the corresponding probe. Most preferably single-stranded DNA is obtained through denaturation of the amplification product, most preferably, through heat-denaturation. The probe specifically hybridises to the region in the product complementary to the 5' tag sequence of the specific ARMS primer. Preferably, the probes are comprised in a microarray, and most preferably, the probes are immobilized on a solid support.

### Definitions:

"Primer" means "primer of a nucleic acid amplification reaction".
Throughout the present disclosure, unless otherwise stated, the term "ARMS primers" refers to the BRAF and PI3K mutation-specific primers designed in their 3' end in accordance with the ARMS amplification method, and comprising a non-target specific 5'-tag sequence; and the term "amplification primers" refers to the primers that combine with the ARMS primers for target DNA amplification.

The ARMS primers used in the different aspects of the invention can be represented as displayed in Table 1 above, wherein the characteristics of the 5' tag sequence or tag are also indicated.
The tag confers probe specificity to the amplification product. Preferably, the 5' tag may be selected from one of the following sequences: SEQ ID Nº 6, SEQ ID Nº 7, SEQ ID Nº 22, SEQ ID Nº 23, SEQ ID Nº 24 and SEQ ID Nº 25.

A particular embodiment of these aspects of the invention, consists on amplification of one or more BRAF or PI3K mutations with one or more ARMS primers of the group of SEQ ID Nº 3, SEQ ID Nº 4, SEQ ID Nº 16, SEQ ID Nº 17, SEQ ID Nº 18 and SEQ ID Nº 19:
**GATTAGCGCAGTGCACTAC**GGTGATTTTGGTCTAGCTTCAGA (SEQ ID Nº 3)(BRAF V600E)
**AGATCGTTATCAATCGCAT**GGTGATTTTGGTCTAGCTACTAA (SEQ ID Nº 4)(BRAF V600K)
**AGACCTTAGCATAGCTT**AAGCAATTTCTACACGAGATCCTCTGTCTA (SEQ ID Nº 16)(PI3K E542K)
**ACTATAGCCGAGTACGGC**CCTCTCTCTGAAATCAGTGAT (SEQ ID Nº 17)(PI3K E545D)
**TAACTGGCTATCCGGAG**GATCCTCTCTCTGAAATCAGTA (SEQ ID Nº 18)(PI3K E545K)
**CGATATGATATGCTAGTT**GAAACAAATGAATGATGCTCGT (SEQ ID Nº 19)(PI3K H1047R).
Nucleotides in bold correspond to the 5' tag.

As amplification primer, any possible primer which, when used in combination with one or more of the ARMS primers of above for amplification, produces amplification products of 1000 bp or shorter, might used.

In a preferred embodiment, the amplification primer combined with the ARMS primers of above produces amplification products of 500 bp or shorter, in another preferred embodiment of around 200 bp, and in a most preferred embodiment, of between 200 bp and 100 bp. Preferably, the amplification primer has a length of from 18 to 24 nucleotides, and most preferably, of from 21 and 22 nucleotides.

Preferably, an amplification primer comprising SEQ ID Nº 5, and most preferably, a primer consisting of sequence SEQ ID Nº 5, is used in combination with one or more of the following ARMS primers:
- A primer of from 36 to 50 nucleotides, comprising a 3' target specific sequence selected from SEQ ID Nº 1 and SEQ ID Nº 2,
   the primer further comprising a non-target specific 5' tag sequence of from 15 to 20 nucleotides; as well as
- Primers of SEQ ID Nº 3 and SEQ ID Nº 4.
   Also, preferably, an amplification primer comprising SEQ ID Nº 20, and most preferably, an amplification primer consisting of sequence SEQ ID Nº 20, is used in combination with any of the following ARMS primers:
- A primer of from 36 to 50 nucleotides, comprising in its 3' position a target specific sequence selected from SEQ ID Nº 12, SEQ ID Nº 13 and SEQ ID Nº 14, the primer further comprising in its 5' end a non-target specific tag sequence of from 15 to 20 nucleotides; as well as
- Primers of SEQ ID Nº 16, SEQ ID Nº 17 and SEQ ID Nº 18.
   Also, preferably, an amplification primer comprising SEQ ID Nº 21, and most preferably, an amplification primer consisting of sequence SEQ ID Nº 21, is used in combination with any of the following ARMS primers:
- A primer of from 36 to 50 nucleotides, comprising in its 3' position a target specific sequence SEQ ID Nº 15, the primer further comprising in its 5' end a non-target specific tag sequence of from 15 to 20 nucleotides; as well as
- A primer of SEQ ID Nº 19.

In a preferred aspect, any specific detection probe has a length of from 15 to 45 nucleotides, the region which specifically hybridizes with the corresponding ARMS product in the region corresponding to the 5'-tag sequence being of between 15 and 20 nucleotides. Each probe may also comprise additional nucleotides, either in the 5' and/or in the 3' end, up to a total probe length of from 15 to 45 nucleotides.

Preferably, the specific probes for detection of BRAF mutations V600E and V600K, or of any of the PI3K mutations E542K, E545D, E545K and H1047R, comprise the nucleotide sequences of SEQ ID Nº 6, SEQ ID Nº 7, SEQ ID Nº 22, SEQ ID Nº 23, SEQ ID Nº 24 and SEQ ID Nº 25, respectively.

One or more different probes may be used for detecting each BRAF or PI3Kmutation.

The most preferred probes for each of the mutations are displayed in Table 2.

| Mutation | Probe sequence | SEQ ID Nº |
|---|---|---|
| BRAF V600E | CGGGTTACCCGGGAGTCTCGATTAGCGCAGTGCACTAC | **8** |
| BRAF V600K | AGATCGTTATCAATCGCATGGTGAT | **9** |
| BRAF V600K | CGGGTTACCCGGGAGATCGTTATCAATCGCAT | **10** |
| BRAF V600K | CGGGTTACCCGGGAGTCTCAGATCGTTATCAATCGCAT | **11** |
| PI3K E542K | CGGGTTACCCGGGAGTCTCAGACCTTAGCATAGCTT | **26** |
| PI3KE545D | CGGGTTACCCGGGACTATAGCCGAGTACGGC | **27** |
| PI3KE545K | CGGGTTACCCGGGAGTCTCTAACTGGCTATCCGGAG | **28** |
| PI3KH1047R | CGATATGATATGCTAGTT | **29** |

In particular, the probes are immobilized on a microarray. A microarray is a collection of microscopic oligonucleotide spots. A DNA microarray (also commonly known as gene chip, DNA chip, or biochip) is a collection of microscopic DNA spots attached to a solid surface. Probes are synthesized and then attached via surface engineering to a solid surface by a covalent bond to a chemical matrix (via epoxy-silane, amino-silane, lysine, polyacrylamide or others). Solid surfaces are known in the art and include microscopic beads as well as solid supports.

In particular, the probes of the present invention may be immobilized on a solid support.

Thereby, further to the mixture for amplification, the kit of the present invention comprises a microarray wherein one or more probes that specifically hybridize to one or more of the products of the BRAF or PI3Kmutations are immobilised.

One or more controls may be included in the methods and kit of the present invention. Preferably, a pair of amplification primers corresponding to any constitutive and ubiquitous human gene known to the skilled person, may be included in the amplification mixture. Most preferably, primers corresponding to β-actin gene are used. Amplification with such a pair of primers allows to confirm the correct extraction of the nucleic acid present in the test sample, and constitutes the "Endogenous control" or "Extraction control". Additionally, a DNA plasmid and a pair of primers with ability to amplify it, are preferably included in the amplification mixture, and constitute the "Amplification control" or "Internal control". Preferably, the microarray may comprise one or more control probes with ability to hybridize to corresponding control DNA sequences, in particular, to the products of the extraction and amplification controls. Preferably, the kit of the present invention further comprises reagents for the visualization of the hybridisation between any amplification product and the microarray of probes.

Another aspect of the present invention corresponds to one or more ARMS primers of a length of from 36 to 50 nucleotides, characterised in that each primer comprises, in its 3' end, a BRAF target specific sequence selected from SEQ ID Nº 1 and SEQ ID Nº 2, or a PI3K target specific sequence selected from SEQ ID Nº 12, SEQ ID Nº 13, SEQ ID Nº 14 and SEQ ID Nº 15, each primer further comprising a non-target specific tag sequence in its 5' end, of from 15 to 20 nucleotides. Preferably, the tags in the 5' position of the ARMS primers contain 15, 16, 17, 18, 19 or 20 nucleotides. Specially preferred ARMS primers are those selected from the group comprising SEQ ID Nº SEQ ID Nº 3, SEQ ID Nº 4, SEQ ID Nº 16, SEQ ID Nº 17, SEQ ID Nº 18 and SEQ ID Nº 19.

Yet another aspect of the present invention corresponds to the use of the methods described herein, or of the kit described herein, or of the primers described herein, for diagnosis and/or prognosis of a pathological condition in a patient, in particular, of cancer, as well as for the prediction of response of a patient to therapy with anti-EGFR antibodies.

In a preferred embodiment, types of test samples that can be processed within the present invention are swabs, paraffin-embedded biopsies, blood, sputum, colonic lavage, bronchial lavage, as well as saline, plasma, and cerebral spinal fluid, and any other body fluid, or tissue obtained from an individual. The individual is human.

The test sample may equally be a nucleic acid sequence corresponding to the sequence present in the test sample. All or a part of the region of interest present in the nucleic acid sample may be amplified using any convenient technique such as PCR, before its use in the method of the invention.

In another preferred embodiment, extraction of genetic material can be carried out both by automatic as well as by manual extraction techniques of the state of the art.

A specially preferred DNA extraction method from tissues and other samples, is QIAGEN's EZ1® DNA Tissue Kit. Also preferred is QIAGEN's product AIIPrep® DNA/RNA FFPE, which is intended for simultaneous purification of genomic DNA and total RNA from formalin-fixed, paraffin-embedded tissue sections. Also, any other techniques and methods for manual or automatic processing of samples to extract DNA and other nucleic acids, the skilled person may be aware of, may be used within the present invention.

In a preferred embodiment of the present invention, the vessel wherein the method of the present invention takes place comprises, further to the one or more ARMS primers and the one or more amplification primers, other components for amplification of the DNA present in the sample. In particular, appropriate nucleotide triphosphates, such as dATP, dCTP, dGTP, dTTP, and a suitable enzyme for polymerisation are also included in the mixture of amplification reagents.

Any convenient enzyme for polymerisation may be used. In particular, any DNA polymerase with ability to discriminate between normal and mutant template sequences to any significant extent. Examples of convenient enzymes include thermostable enzymes which have no significant 3'-5'exonuclease activity, and with polymerization rates of around 10 nucleotides/ second, thereby yielding amplification fragments of 600-1000bp in standard extension steps. Preferably QIAGEN's HotStarTaq DNA Polymerase may be used. Any other enzyme with these characteristics known in the state of the art, may also be used. For instance, "Ampli Taq Gold" DNA polymerase of PE Applied Biosystems.

ARMS amplification of the different mutations of the present invention may be carried out either individually, or in a Multiplex amplification reaction of two or more of the mutations. In both cases, QIAGEN Multiplex PCR Kit is most preferably used for ARMS amplification, within the methods and kits of the present invention.

The method of the present invention can be carried out in one or more reaction vessels, each vessel comprising at least one ARMS primer, and at least one amplification primer, together with other reagents for amplification.

The agent for polymerisation and the appropriate conditions can be selected by the skilled person. Standard thermal cycling conditions may be used for amplification of the mutations according to the present invention.

Thermal cycling conditions that have proven to work particularly well with samples are as follows:

| **NUMBER OF CYCLES** | **TEMPERATURE** | **TIME** |
|---|---|---|
| 1 cycle | 95ºC | 15' |
| 40 cycles | 94ºC | 15" |
| | 62ºC | 60" |
| 1 cycle | 72ºC | 10' |
| 1 cycle | 4ºC | Forever |

Any possible combination of the ARMS primers of the present invention may be used for the Multiplex detection of one or more of the BRAF or PI3K mutations present in a sample.

Thus, the reaction vessel wherein the method of the present invention takes place, may comprise 1, 2, 3, 4, 5 or 6 of the ARMS primers of Table 1, or of the primers selected from SEQ ID Nº 3, SEQ ID Nº 4, SEQ ID Nº 16, SEQ ID Nº 17, SEQ ID Nº 18 and SEQ ID Nº19, as well as one or more amplification primers that might be combined with the primers of above for amplification of one or more of the BRAF and PI3K mutations present in a sample.

Additional primer pairs, preferably those corresponding to extraction and/or internal controls, and/or pairs of primers for amplification of other mutations, may also be included in the amplification mixture.

Specially preferred combinations of ARMS primers are primer mixtures comprising:
- ARMS Primers of SEQ ID Nº 3 (BRAF V600E) and SEQ ID Nº 4 (BRAF V600K) (Components of Amplification Mixture 1);
- ARMS Primers of SEQ ID Nº16 (PI3K E542K) and SEQ ID Nº 19 (PI3K H1047R) (Components of Amplification Mixture 2);
- ARMS Primers of SEQ ID Nº 17 (PI3K E545D) and SEQ ID Nº 18 (PI3K E545K) (Components of Amplification Mixture 3);
- ARMS Primers of SEQ ID Nº 16, SEQ ID Nº 17, SEQ ID Nº 18 and SEQ ID Nº 19 (PI3K E542K, PI3K E545D, PI3K E545K and PI3K H1047R, respectively) (Components of Amplification Mixture 4).

Specially preferred combinations of ARMS and amplification primers are primer mixtures comprising:
- ARMS Primer of SEQ ID Nº 3 (BRAF V600E), ARMS Primer of SEQ ID Nº 4 (BRAF V600K), Amplification Primer of SEQ ID Nº 5 (Common Amplification "Primer" to ARMS Primers of SEQ ID Nº 3 and SEQ ID Nº 4) (Components of Amplification Mixture 1);
- ARMS Primers of SEQ ID Nº 16 (PI3K E542K) and SEQ ID Nº 19 (PI3K H1047R), Amplification primers of SEQ ID Nº 20 and SEQ ID Nº 21 (Amplification "Primers" corresponding to ARMS Primers of SEQ ID Nº 16 and SEQ ID Nº 19, respectively) (Components of Amplification Mixture 2);
- ARMS Primers of SEQ ID Nº 17 (PI3K E545D) and SEQ ID Nº 18 (PI3K E545K) and Amplification Primer of SEQ ID Nº 20 (Common Amplification "Primer" to ARMS Primers of SEQ ID Nº 17 and SEQ ID Nº 18) (Components of Amplification Mixture 3);
- ARMS Primers of SEQ ID Nº 16 (PI3K E542K), SEQ ID Nº 17 (PI3K E545D), SEQ ID Nº 18 (PI3K E545K) and SEQ ID Nº 19 (PI3K H1047R), Amplification Primer of SEQ ID Nº 20 (Common Amplification "Primer" to ARMS Primers of SEQ ID Nº 16, SEQ ID Nº 17 and SEQ ID Nº 18) and SEQ ID Nº 21 (Amplification "Primer" corresponding to ARMS Primer of SEQ ID Nº 19) (Components of Amplification Mixture 4).

Remaining components of the different Amplification Mixtures include a DNA Polymerase, dNTPs and any other necessary component for amplification to take place. Further, the different Amplification mixtures may additionally contain forward and reverse primers for amplification of the Internal and Extraction controls, as well as pairs of primers for amplification of other mutations.

The method of the present invention has proven to easily detect amounts of BRAF and PI3K mutated DNA of from 1 ng to 1 µg. In particular, the method of the present invention has shown to detect without problem, 5 µl of a cell line with 200 ng/µl BRAF or PI3K mutated DNA, as well as serial dilutions of the former up to 0.2 ng/µl. The limit of detection of the kit of the present invention is 1,000 copies of mutant BRAF or PI3K in a 5 µl sample. The sensitivity of detection value of the kit for the different BRAF or PI3K mutations is of 1%. As regards diagnostic parameters, the Diagnostic Sensitivity value of the kit for the BRAF and PI3K mutations is higher than 98%.

A label may be introduced in the DNA amplification product during ARMS amplification to allow further detection; in particular, a label that provides a signal that may be detected by colorimetric methods, by fluorescent methods, or by any labelling method known in the art. The label can be radioactive, chemiluminescent, luminescent and fluorescent agents. In a preferred aspect, the label that is used is biotin. However, any other kind of label known in the art may be used (eg. digoxigenin). In a preferred aspect, at least one of the primers used is labelled at the 5' end with biotin. Preferably, the amplification primer is labelled. Furthermore, labelling of amplified DNA may alternatively be achieved by adding modified nucleotides bearing a label (e.g. biotinylated or digoxigenin dUTP derivatives) to the PCR mixture. In certain embodiments, radioactive labels may be used, as well as fluorophores.

Alternative methods known to the skilled person, that may enable detection of the interaction between any amplification product and its corresponding probe, including methods that imply labelling of the probe, may be used.

In a preferred embodiment of the present invention, amplification products, previously denatured, are incubated with target-specific probes that hybridize with the amplification products at least in the region corresponding to the 5' tag of nucleotides provided by the specific primer.

Preferably, denaturing of amplified DNA can be performed by heating. Other ways to prepare single-stranded DNA after amplification may be used as well; for example, chemical means.

In a preferred aspect, the test sample comprising nucleic acids to be analysed, is divided in two or more aliquots, wherein:
- one of the aliquots is subjected to amplification with a mixture comprising the primers of SEQ ID Nº 3, SEQ ID Nº 4 and SEQ ID Nº 5 (Amplification Mixture 1);
- another aliquot is subjected to amplification with a mixture comprising the primers of SEQ ID Nº 16, SEQ ID Nº 19, SEQ ID Nº 20 and SEQ ID Nº 21 (Amplification Mixture 2), and
- another aliquot is subjected to amplification with a mixture comprising the primers of SEQ ID Nº 17, SEQ ID Nº 18 and SEQ ID Nº 20 (Amplification Mixture 3),
   or else,
- one of the aliquots is subjected to amplification with Mixture 1 of above; and
- another aliquot is subjected to amplification with a mixture comprising the primers of SEQ ID Nº 16, SEQ ID Nº 17, SEQ ID Nº 18, SEQ ID Nº 19, SEQ ID Nº 20 y SEQ ID Nº 21 (Amplification Mixture 4).

The method further comprises denaturation of any amplification product obtained, and its subsequent hybridisation with a microarray comprising one or more of the probes selected from SEQ ID Nº 8, SEQ ID Nº 9, SEQ ID Nº 10, SEQ ID Nº 11, SEQ ID Nº 26, SEQ ID Nº 27, SEQ ID Nº 28 and SEQ ID Nº 29.

In a preferred embodiment of the present invention, the probes for detection of the amplification products are provided in a microarray. Microarray technology enables simultaneous detection of different amplification products, corresponding to one or more mutations present in a sample, in the presence of any controls needed to ensure reliability of the results.

In a preferred embodiment of the present invention, single-stranded DNA obtained from one or more amplification products, is incubated with a plurality of target-specific probes provided on a microarray. At least one, but preferably more than one probe with ability to hybridise with each target sequence, are provided on the microarray. In certain embodiments of the invention, the single-stranded DNA may be incubated with target-specific probes provided in solution; however, it is preferred that the probes are arranged on a microarray.

Described herein are probes contained in a microarray, which may be placed on a slide or contained in a reaction vessel, which is then called an array vessel. Array vessels may have different formats of presentation, including individual array vessels, such as wells or tubes, or sets of array vessels arranged in strips of wells or tubes, or flat plates. Usually, plates consist of sets of strips of array vessels. Thus, a microarray of the present invention may be contained in an individual array vessel. Alternatively, two or more microarrays may be contained in a strip of vessels. In a preferred embodiment, the strip of vessels is constituted by 8 vessels. Further, three or more array vessels may be arranged in a set of strip of vessels. In another preferred embodiment, the set of strip of vessels is a microtiter plate. In yet another preferred embodiment, the microtiter plate is constituted by 96 array vessels.

In preferred embodiments, the probes of the microarray may be immobilised on a solid support wherein this solid support can be the bottom of an array vessel or a different solid support attached to the bottom of an array vessel. This means that the surface of the microarray may be the flat bottom of the array vessel. Alternatively, the surface of the microarray may be a solid support attached to the bottom of the array vessel.

In an embodiment of the present invention, the reaction vessel has a typical size for a laboratory reaction vessel. Typical filling volumes lie in the range of 100 µl to 2.5 ml, but can also be lower or higher in special embodiments. The reaction vessel may have a normal filling volume for a standard Eppendorf tube of up to 1.5 ml. Further preferred filling volumes are up to 0.4 ml, up to 0.5 ml, up to 0.7 ml, up to 1.0 ml or up to 2.0 ml. Due to the labelling of the amplified DNA, wherever sample molecules interact with probe molecules on the surface of the microarray, a reporter reagent binds the label and produces visible signals which may be detected by a detection device.

The interacting probe and sample molecules are identified by the location of the signal on the surface of the microarray. In the particular case where sample amplification products are labelled with biotin, the reporter agent can be horseradish peroxidase covalently joined to streptavidin. The latter binds specifically to biotin, and the peroxidase triggers the precipitation of substrates like tetramethylbenzidine (TMB). Any other reaction that results in a precipitate on array elements, and that can be used to detect the interaction between target and probe molecules according to the present invention may equally be used. Any other detection method known in the state of the art, such as fluorescence, may be used for detection of the interaction between amplification products and corresponding probes. The method will be dependent on the exact labelling of the amplification products.

The probes of the present invention can be obtained by different methods, such as chemical synthesis (e. g. by the conventional phosphotriester method) or genetic engineering techniques, for example by molecular cloning of recombinant plasmids in which corresponding nucleotide sequences have been inserted and can be latter obtained by digestion with nucleases.

Individual probes or mixtures of probes specific for each mutation, may be immobilized in a single location of the solid support, in two distinct locations of the solid support and in three or more distinct locations of the solid support.

Additionally, one or more control probes are also provided in distinct locations.

In a preferred embodiment, visualization of the interactions between amplification products and their corresponding specific or control probes, consists of the following steps:
- First, the image of the array is captured using an optical device;
- Then, the image is analysed;
- Finally, a report containing an interpretation of the result is provided.

Preferably, the image is analysed by means of appropriate software. Any device suitable for this processing can be used.

Detection of the BRAF and PI3K mutations with the method of the present invention is compatible with detection of other mutations in these same genes, as well as of mutations in other genes relevant in cancer.

As a matter of fact, the method of the present invention can be combined with other methods of detection of mutations, either in the BRAF and PI3K genes and/or in any other genes relevant in cancer, for performing a thorough diagnosis and/or prognosis of cancer.

The method of detection of the 2 BRAF mutations V600E and V600K, or of the 4 PI3K mutations E542K, E545D, E545K and H1047R of the present invention, can be applied to any pathology and to any sample suspicious of correlating with BRAF or PI3K mutations.

The examples provided below merely illustrate the invention and in no way limit the scope of the accompanying claims.

### Examples.

### Example 1.

The following mixture of reagents was prepared for Multiplex ARMS amplification of the BRAF mutations V600E and V600K in samples/ cell lines/ clones:

| **BRAF ARMS Multiplex Reagents** **(Amplification Mixture 1)** | **Stock Concentration** **(µM)** | **µl/ tube** |
|---|---|---|
| 2X QIAGEN Multiplex PCR Master Mix | | 25 |
| ARMS primer, SEQ ID Nº 3 | 40 | 0.25 |
| ARMS primer, SEQ ID Nº 4 | 40 | 0.25 |
| Amplification primer, SEQ 10 Nº 5 (Biotin labeled) | 40 | 1.25 |
| H2O/ IC and/or EC reagents | | Up to 45 µl |

Next, 5 µl of a total eluate of 30 µl obtained from paraffin-embedded tissue sections (sample), or from different cell lines or clones, were added up to a final reaction volume of 50 µl.

The thermal cycling conditions of the PCR were:

| **NUMBER OF CYCLES** | **TEMPERATURE** | **TIME** |
|---|---|---|
| 1 cycle | 95ºC | 15' |
| 40 cycles | 94ºC | 15" |
| | 62ºC | 60" |
| 1 cycle | 72ºC | 10' |
| 1 cycle | 4ºC | Forever |

Results of the BRAF ARMS Multiplex amplification are displayed in Figure 2 and Figure 3.

The different ARMS products of the samples/ clones or cell lines indicated in Table 3 of Figure 2, were visualized in a 2% agarose gel, wherein the products of the different amplification reactions were analysed.

The length of the different amplification products are as follows:
- Length of the mutation-specific band corresponding to mutations V600E and V600K: 144bp;
- Length of the IC band: 101 bp (amplification product of the beta-actin gene);
- Length of the EC band: 112bp (amplification product of plasmid ppg25).

In spite of the fact that the length of the different amplification products is similar, differences can be appreciated between the wells that contain some mutation-specific band together with the control bands, and the wells that just contain the control bands. This difference corresponds to the mutation-specific amplification product.

In these experiments it has been checked that ARMS amplification only takes place when the sample subjected to amplification, is that of a sample/ clon/ cell line, corresponding to one of the mutations for which ARMS primers have been included in the mixture of reagents used for Multiplex amplification.

It has been confirmed by DNA sequencing that the ARMS products obtained in each well really result from the specific amplification of the sample/ clon/ cell line, with the corresponding ARMS primer. ARMS amplification in each Multiplex is specific.

It has been confirmed that small variations in the number of amplification cycles (2-3 extra or less amplification cycles) do not modify the results obtained.

The different Multiplex ARMS amplification products where denatured and hybridized with microarrays of probes. Visualization of the corresponding results is displayed in Figure 3.

### Example 2.

Composition of Amplification Mixtures 2 and 3 corresponding to PI3K are displayed below. Remaining reaction conditions are the same as those displayed in Example 1 for BRAF.

| **PI3K ARMS Multiplex Reagents** **(Amplification Mixture 2)** | **Stock Concentration** **(µM)** | **µl/ tube** |
|---|---|---|
| 2X QIAGEN Multiplex PCR Master Mix | | 25 |
| ARMS primer, SEQ ID Nº 16 | 40 | 0.25 |
| Amplification primer, SEQ ID Nº 20, (Biotin labeled) | 40 | 0.50 |
| ARMS primer, SEQ ID Nº19 | 40 | 0.25 |
| Amplification primer, SEQ ID Nº21, (Biotin labeled) | 40 | 0.50 |
| H2O/ IC and/or EC reagents | | Up to 45 µl volume |

| **PI3K ARMS Multiplex Reagents** **(Amplification Mixture 3)** | **Stock Concentration** **(µM)** | **µl/ tube** |
|---|---|---|
| 2X QIAGEN Multiplex PCR Master Mix | | 25 |
| ARMS primer, SEQ ID Nº17 | 40 | 0.25 |
| ARMS primer, SEQ ID Nº18 | 40 | 0.25 |
| Amplification primer, SEQ ID Nº 20, (Biotin labeled) | 40 | 0.50 |
| H2O/ IC and/or EC reagents | | Up to 45 µl volume |

Results corresponding to 5*µ*l of cell line HTC 116 (0.2ng/*µ*l) which contains PI3K mutation H1047R (a), or of 5*µ*l of a clinical sample containing DNA at a concentration of 20ng/*µ*l (b), after their respective amplifications with reagents of Amplification Mixture 2, and the subsequent hybridization of the obtained products with a probe microarray, and subsequent visualization, are displayed in Figures 4a) and 4b), respectively.

### Example 3:

Inclusion within the Amplification Mixtures, of the forward and reverse primers that are necessary for Internal Control and Extraction Control amplification, as well as of a plasmid such as pBSK with an insert, also necessary for Internal Control amplification, at the concentrations displayed below, does not alter the obtained results:

| **ARMS Multiplex Reagents** | **Stock Concentration** **(µM)** | **µl/ tube** |
|---|---|---|
| EC forward primer | 40 | 0.13-0.25 |
| EC reverse primer (Biotin labeled) | 40 | 0.13-0.25 |
| IC forward primer | 40 | 0.13-0.25 |
| IC reverse primer (Biotin labeled) | 40 | 0.13-0.25 |
| Plasmid pBSK with an insert | 10e4 copies | 0.25-0.5 |

| | | |
|---|---|---|
| IC: Internal Control; EC: Extraction Control. | | |

## Claims

1. A method for detecting one or more BRAF mutations selected from V600E and V600K, or one or more PI3K mutations selected from E542K, E545D, E545K and H1047R, wherein the method comprises the steps of:
- subjecting a test sample comprising nucleic acids to amplification with an amplification mixture comprising one or more ARMS primers, **characterised in that** each ARMS primer has a total length of from 36 to 50 nucleotides, and comprises a 3' target specific sequence selected from SEQ ID Nº1, SEQ ID Nº 2, SEQ ID Nº 12, SEQ ID Nº 13, SEQ ID Nº 14 and SEQ ID Nº 15, respectively,
each ARMS primer further comprising a different non-target specific 5' tag sequence of from 15 to 20 nucleotides,
the amplification mixture further comprising one or more amplification primers; and
- detecting any amplification product obtained, through hybridisation of any such product with one or more probes, wherein each probe specifically hybridises with the region in the amplification product corresponding to the 5' tag sequence of the corresponding ARMS primer.

2. The method of claim 1 wherein one or more ARMS primers are selected from SEQ ID Nº 3, SEQ ID Nº 4, SEQ ID Nº 16, SEQ ID Nº 17, SEQ ID Nº 18 and SEQ ID Nº 19.

3. The method of claims 1 and 2 wherein the one or more amplification primers which are combined with one or more ARMS primers comprise from 18 to 24 nucleotides, and are such that, when combined with the one or more ARMS primers, amplification results in one or more products of 1000 bp or shorter.

4. The method of any of claims 1 to 3, wherein the amplification primer is:
- A primer comprising or consisting of SEQ ID Nº 5, for amplification with one or more of the following ARMS primers:
- Primers of from 36 to 50 nucleotides, comprising a 3' target specific sequence selected from SEQ ID Nº 1 and SEQ ID Nº 2, the primers further comprising a non-target specific 5' tag sequence of from 15 to 20 nucleotides; and
- Primers of SEQ ID Nº 3 and SEQ ID Nº 4.
- A primer comprising or consisting of SEQ ID Nº 20, for amplification with one or more of the following ARMS primers:
- Primers of from 36 to 50 nucleotides, comprising a 3' target specific sequence selected from SEQ ID Nº 12, SEQ ID Nº 13 y SEQ ID Nº 14, the primers further comprising a non-target specific 5' tag sequence of from 15 to 20 nucleotides; and
- Primers of SEQ ID Nº 16, SEQ ID Nº 17 and SEQ ID Nº 18.
- A primer comprising or consisting of SEQ ID Nº 21, for amplification with one or more of the following ARMS primers:
- Primers of from 36 to 50 nucleotides, comprising a 3' target specific sequence SEQ ID Nº 15, the primers further comprising a non-target specific 5' tag sequence of from 15 to 20 nucleotides; and
- A Primer of SEQ ID Nº 19.

5. The method of any of claims 1 to 4 wherein the amplification mixture comprises a DNA Polymerase and dNTPs.

6. The method of any of claims 1 to 5 wherein one, two, three or four of the following primer combinations are used:
- SEQ ID Nº 3, SEQ ID Nº 4 and SEQ ID Nº 5;
- SEQ ID Nº 16, SEQ ID Nº 19, SEQ ID Nº 20 and SEQ ID Nº 21;
- SEQ ID Nº 17, SEQ ID Nº 18 and SEQ ID Nº 20;
- SEQ ID Nº 16, SEQ ID Nº 17, SEQ ID Nº 18, SEQ ID Nº 19, SEQ ID Nº 20 and SEQ ID Nº 21.

7. The method of any of claims 1 to 6, wherein one or more probes with which the amplification products are hybridised have a length of from 15 to 45 nucleotides, and comprise a sequence selected from the group of SEQ ID Nº 6, SEQ ID Nº 7, SEQ ID Nº 22, SEQ ID Nº 23, SEQ ID Nº 24 and SEQ ID Nº 25.

8. The method of any of claims 1 to 7 wherein one or more probes are selected from SEQ ID Nº 8, SEQ ID Nº 9, SEQ ID Nº 10, SEQ ID Nº 11, SEQ ID Nº 26, SEQ ID Nº 27, SEQ ID Nº 28 and SEQ ID Nº 29.

9. A kit for detecting one or more BRAF mutations selected from V600E and V600K, or one or more PI3K mutations selected from E542K, E545D, E545K and H1047R in a test sample comprising nucleic acid, wherein said kit comprises one or more mixtures of reagents for nucleic acid amplification, each mixture comprising:
- one or more ARMS primers **characterised in that** each primer has a length of from 36 to 50 nucleotides, comprising a 3' target specific sequence selected from SEQ ID Nº 1, SEQ ID Nº 2, SEQ ID Nº 12, SEQ ID Nº 13, SEQ ID Nº 14 and SEQ ID Nº 15, respectively,
each primer further comprising a different non-target specific 5' tag sequence of from 15 to 20 nucleotides, and
- one or more amplification primers,
the kit further comprising a microarray wherein one or more probes are immobilised, each probe specifically hybridising to the region in the corresponding ARMS product complementary to the 5' tag sequence of the corresponding ARMS primer.

10. The kit of claim 9 wherein one or more probes have a length of from 15 to 45 nucleotides, and comprise a sequence selected from SEQ ID Nº 6, SEQ ID Nº 7, SEQ ID Nº 22, SEQ ID Nº 23, SEQ ID Nº 24 and SEQ ID Nº 25.

11. The kit of claims 9 and 10 comprising one or more of the following amplification mixtures:
- Amplification mixture comprising primers of SEQ ID Nº 3, SEQ ID Nº 4 y SEQ ID Nº 5;
- Amplification mixture comprising primers of SEQ ID Nº 16, SEQ ID Nº 19, SEQ ID Nº 20 and SEQ ID Nº 21;
- Amplification mixture comprising primers of SEQ ID Nº 17, SEQ ID Nº 18 and SEQ ID Nº 20; and
- Amplification mixture comprising primers of SEQ ID Nº 16, SEQ ID Nº 17, SEQ ID Nº 18, SEQ ID Nº 19, SEQ ID Nº 20 and SEQ ID Nº 21;
the kit further comprising
- A microarray comprising one or more of the probes selected from SEQ ID Nº 8, SEQ ID Nº 9, SEQ ID Nº 10, SEQ ID Nº 11, SEQ ID Nº 26, SEQ ID Nº 27, SEQ ID Nº 28 y SEQ ID Nº 29.

12. An amplification method of a nucleic acid corresponding to one or more BRAF mutations selected from V600E and V600K, or to one or more PI3K mutations selected from E542K, E545D, E545K and H1047R, wherein the method comprises contacting a sample containing nucleic acids with an amplification mixture comprising one or more ARMS primers **characterised in that** each ARMS primer has a total length of from 36 to 50 nucleotides, and comprises a 3' target specific sequence selected from SEQ ID Nº 1, SEQ ID Nº 2, SEQ ID Nº 12, SEQ ID Nº 13, SEQ ID Nº 14 and SEQ ID Nº 15, respectively,
each ARMS primer further comprising a different non-target specific 5' tag sequence of from 15 to 20 nucleotides,
the amplification mixture further comprising one or more amplification primers.

13. An ARMS primer of a length of from 36 to 50 nucleotides, **characterized in that** said primer comprises, in its 3' end, a BRAF target specific sequence selected from SEQ ID Nº 1 and SEQ ID Nº 2, or a PI3K target specific sequence selected from SEQ ID Nº 12, SEQ ID Nº 13, SEQ ID Nº 14 and SEQ ID Nº 15,
and a non-target specific tag sequence of from 15 to 20 nucleotides, in its 5' end.

14. The primer of claim 13, selected from the group comprising SEQ ID Nº 3, SEQ ID Nº 4, SEQ ID Nº 16, SEQ ID Nº 17, SEQ ID Nº 18 and SEQ ID Nº 19.

15. Use of the method of detection of BRAF and PI3K mutations of claims 1 to 8, or of the kit of claims 9 to 11, or of the amplification method of claim 12, or of the primers of claims 13 and 14, for diagnosing and/ or prognosing a pathologic condition in a patient, or for predicting response of a patient to therapy with anti-EGFR antibodies.


SEQUENCE LISTING

<110> GENOMICA S.A.U.

<120> Method for detection of BRAF and PIK3CA mutations

<130> BRAF PI3K EP2012

<160> 29

<170> PatentIn version 3.3

<210> 1
<211> 23
<212> DNA
<213> Artificial Sequence
<220>
<223> 3'sequence of ARMS primer for amplification of BRAF mutation
V600E, the sequence being target-specific

<400> 1
ggtgattttg gtctagcttc aga                                              23

<210> 2
<211> 23
<212> DNA
<213> Artificial Sequence
<220>
<223> 3'sequence of ARMS primer for amplification of BRAF mutation
V600K, the sequence being target-specific

<400> 2
ggtgattttg gtctagctac taa                                              23

<210> 3
<211> 42
<212> DNA
<213> Artificial Sequence
<220>
<223> ARMS primer for amplification of BRAF mutation V600E

<400> 3
gattagcgca gtgcactacg gtgattttgg tctagcttca ga                         42

<210> 4
<211> 42
<212> DNA
<213> Artificial Sequence
<220>
<223> ARMS primer for amplification of BRAF mutation V600K

<400> 4
agatcgttat caatcgcatg gtgattttgg tctagctact aa                         42

<210> 5
<211> 22
<212> DNA
<213> Artificial Sequence
<220>
<223> Amplification primer to be used in combination with any of
ARMS primers comprising SEQ ID Nº 1 and 2, or

ARMS primers consisting of SEQ ID Nº 3 and 4.

<400> 5
ggccaaaaat ttaatcagtg ga                                                22

<210> 6
<211> 19
<212> DNA
<213> Artificial Sequence
<220>
<223> 5\222 sequence of ARMS primer for amplification of BRAF mutation
V600E, the sequence being non-target-specific.
This sequence is also present in the detection probe of
BRAF mutation V600E.

<400> 6
gattagcgca gtgcactac                                                    19

<210> 7
<211> 19
<212> DNA
<213> Artificial Sequence
<220>
<223> 5\222 sequence of ARMS primer for amplification of BRAF mutation
V600K, the sequence being non-target-specific.
This sequence is also present in the detection probe of
BRAF mutation V600K.

<400> 7
agatcgttat caatcgcat                                                    19

<210> 8
<211> 38
<212> DNA
<213> Artificial Sequence
<220>
<223> Probe for detection of ARMS amplification product of
BRAF mutation V600E

<400> 8
cgggttaccc gggagtctcg attagcgcag tgcactac                               38

<210> 9
<211> 25
<212> DNA
<213> Artificial Sequence
<220>
<223> Probe for detection of ARMS amplification product of
BRAF mutation V600K

<400> 9
agatcgttat caatcgcatg gtgat                                             25

<210> 10
<211> 32
<212> DNA
<213> Artificial Sequence
<220>
<223> Probe for detection of ARMS amplification product of
BRAF mutation V600K

<400> 10
cgggttaccc gggagatcgt tatcaatcgc at                                     32

<210> 11

<211> 38
<212> DNA
<213> Artificial Sequence
<220>
<223> Probe for detection of ARMS amplification product of
BRAF mutation V600K

<400> 11
cgggttaccc gggagtctca gatcgttatc aatcgcat                                38

<210> 12
<211> 30
<212> DNA
<213> Artificial Sequence
<220>
<223> 3'sequence of ARMS primer for amplification of PIK3CA mutation
E542K, the sequence being target-specific

<400> 12
aagcaatttc tacacgagat cctctgtcta                                         30

<210> 13
<211> 21
<212> DNA
<213> Artificial Sequence
<220>
<223> 3'sequence of ARMS primer for amplification of PIK3CA mutation
E545D, the sequence being target-specific

<400> 13
cctctctctg aaatcagtga t                                                  21

<210> 14
<211> 22
<212> DNA
<213> Artificial Sequence
<220>
<223> 3'sequence of ARMS primer for amplification of PIK3CA mutation
E545K, the sequence being target-specific

<400> 14
gatcctctct ctgaaatcag ta                                                 22

<210> 15
<211> 22
<212> DNA
<213> Artificial Sequence
<220>
<223> 3'sequence of ARMS primer for amplification of PIK3CA mutation
H1047R, the sequence being target-specific

<400> 15
gaaacaaatg aatgatgctc gt                                                 22

<210> 16
<211> 47
<212> DNA
<213> Artificial Sequence
<220>
<223> ARMS primer for amplification of PIK3CA mutation E542K

<400> 16
agaccttagc atagcttaag caatttctac acgagatcct ctgtcta                      47

<210> 17

<211> 39
<212> DNA
<213> Artificial Sequence
<220>
<223> ARMS primer for amplification of PIK3CA mutation E545D

<400> 17
actatagccg agtacggccc tctctctgaa atcagtgat                              39

<210> 18
<211> 39
<212> DNA
<213> Artificial Sequence
<220>
<223> ARMS primer for amplification of PIK3CA mutation E545K

<400> 18
taactggcta tccggaggat cctctctctg aaatcagta                              39

<210> 19
<211> 40
<212> DNA
<213> Artificial Sequence
<220>
<223> ARMS primer for amplification of PIK3CA mutation H1047R

<400> 19
cgatatgata tgctagttga aacaaatgaa tgatgctcgt                             40

<210> 20
<211> 21
<212> DNA
<213> Artificial Sequence
<220>
<223> Amplification primer to be used in combination with any of
ARMS primers comprising SEQ ID Nº 12, 13 and 14, or ARMS primers
consisting of SEQ ID Nº 16, 17 and 18.

<400> 20
acatgctgag atcagccaaa t                                                 21

<210> 21
<211> 21
<212> DNA
<213> Artificial Sequence
<220>
<223> Amplification primer to be used in combination with any of
ARMS primers comprising SEQ ID Nº 15, or ARMS primer consisting of SEQ ID Nº
19.

<400> 21
tggaatccag agtgagcttt c                                                 21

<210> 22
<211> 17
<212> DNA
<213> Artificial Sequence
<220>
<223> 5\222 sequence of ARMS primer for amplification of PIK3CA mutation
E542K
the sequence being non-target-specific.
This sequence is also present in the detection probe of PIK3CA mutation E542K

<400> 22
agaccttagc atagctt                                                      17

<210> 23
<211> 18
<212> DNA
<213> Artificial Sequence
<220>
<223> 5\222 sequence of ARMS primer for amplification of PIK3CA mutation
E545D
the sequence being non-target-specific.
This sequence is also present in the detection probe of PIK3CA mutation E545D

<400> 23
actatagccg agtacggc                                                    18

<210> 24
<211> 17
<212> DNA
<213> Artificial Sequence
<220>
<223> 5\222 sequence of ARMS primer for amplification of PIK3CA mutation
E545K
the sequence being non-target-specific.
This sequence is also present in the detection probe of PIK3CA mutation E545K

<400> 24
taactggcta tccggag                                                     17

<210> 25
<211> 18
<212> DNA
<213> Artificial Sequence
<220>
<223> 5\222 sequence of ARMS primer for amplification of PIK3CA mutation
H1047R,
the sequence being non-target-specific.
This sequence is also present in the detection probe of PIK3CA mutation
H1047R.

<400> 25
cgatatgata tgctagtt                                                    18

<210> 26
<211> 36
<212> DNA
<213> Artificial Sequence
<220>
<223> Probe for detection of ARMS amplification product of
PIK3CA mutation E542K

<400> 26
cgggttaccc gggagtctca gaccttagca tagctt                                36

<210> 27
<211> 31
<212> DNA
<213> Artificial Sequence
<220>
<223> Probe for detection of ARMS amplification product of
PIK3CA mutation E545D

<400> 27
cgggttaccc gggactatag ccgagtacgg c                                     31

<210> 28
<211> 36

<212> DNA
<213> Artificial Sequence
<220>
<223> Probe for detection of ARMS amplification product of
PIK3CA mutation E545K

<400> 28
cgggttaccc gggagtctct aactggctat ccggag                                36

<210> 29
<211> 18
<212> DNA
<213> Artificial Sequence
<220>
<223> Probe for detection of ARMS amplification product of
PIK3CA mutation H1047R

<400> 29
cgatatgata tgctagtt                                                    18